(19) Europäisches Patentamt
European Patent Office
Office européen des brevets

(11) **EP 4 546 363 A1**

(12) **EUROPEAN PATENT APPLICATION**
published in accordance with Art. 153(4) EPC

(43) Date of publication:
**30.04.2025 Bulletin 2025/18**

(21) Application number: **23846947.2**

(22) Date of filing: **25.07.2023**

(51) International Patent Classification (IPC):
*G16H 20/60* (2018.01)  *G16H 50/50* (2018.01)
*G16H 50/20* (2018.01)  *G16H 50/70* (2018.01)
*A61B 5/346* (2021.01)  *G06N 3/045* (2023.01)
*G06N 3/042* (2023.01)  *G06N 3/08* (2023.01)

(52) Cooperative Patent Classification (CPC):
A61B 5/346; G06N 3/042; G06N 3/045; G06N 3/08;
G16H 20/60; G16H 50/20; G16H 50/50;
G16H 50/70

(86) International application number:
**PCT/KR2023/010703**

(87) International publication number:
**WO 2024/025288 (01.02.2024 Gazette 2024/05)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **29.07.2022 KR 20220094655**

(71) Applicant: **Medical AI Co., Ltd.**
**Seoul 06180 (KR)**

(72) Inventor: **KWON, Joonmyoung**
**Seoul 06180 (KR)**

(74) Representative: **Patentanwaltskanzlei**
**Matschnig & Forsthuber OG**
**Biberstraße 22**
**Postfach 36**
**1010 Wien (AT)**

(54) **ELECTROCARDIOGRAM-BASED METHOD FOR RECOMMENDING MENU, AND COMPUTER PROGRAM RECORDED ON RECORDING MEDIUM IN ORDER TO EXECUTE SAME**

(57)     The present invention proposes a method of recommending a menu suitable for a user's real-time health condition based on an electrocardiogram (ECG). The menu recommendation method may include: obtaining an electrocardiogram signal measured for a user; identifying one or more lacked nutrients and one or more surplus nutrients corresponding to the user's health condition by analyzing the electrocardiogram signal using pre-trained artificial intelligence (AI); and setting a menu including at least one food that can supplement the identified lacked nutrients and avoid the identified surplus nutrients. Through this, a user can receive a recommendation for a menu optimized for the user's health condition at that time simply by measuring an electrocardiogram.

FIG. 10

EP 4 546 363 A1

## Description

## Technical Field

The present invention relates to smart health care, and more particularly, to a method of recommending a menu suitable for a user's real-time health condition based on an electrocardiogram (ECG) and a computer program recorded on a storage medium in order to execute the method.

## Background Art

An electrocardiogram is a graphical recording of visual changes in electrical activity generated by the heart muscles. Briefly, as for the conduction system of the heart, the sinoatrial node (the SA node) periodically generates an electrical signal to induce the contraction of the heart. When the electrical signal generated by the sinoatrial node (the SA node) is transmitted through the atrium, the atrioventricular node (the AV node) slightly delays the electrical signal and then transmits it. The electrical signal transmitted from the AV node spreads throughout the ventricle through the bundle of His and Purkinje fibers.

As for the waveforms of an electrocardiogram that can be identified according to the process of conduction of the heart, the P wave can be identified during the process in which as the atrium becomes polarized by the electrical signal generated by the sinoatrial node (the SA node), the heart muscles of the valve contract, and, as the atrium becomes depolarized again, the heart muscles of the valve relax again. The P-Q wave can be identified during the process in which the AV node delays the electrical signal so that the ventricle does not immediately respond when the atrium contracts. The QRS complex can be identified during the process in which the electrical signal delayed by the AV node is transmitted (Q), and thus, the ventricle becomes polarized (R) immediately and then depolarized (S) immediately. The S-T wave can be identified during the resting phase in which the electrical signal does not stimulate the heart so that the ventricle contracts and the blood in the ventricle moves to various parts of the body. Furthermore, the T wave can be identified during the process in which as the ventricle is weakly polarized and then depolarized again, the heart muscles of the ventricle and the valve relax simultaneously. Furthermore, in some cases, the U wave can be identified due to the repolarization of the intraventricular septum.

The order of appearance, shapes, sizes, and intervals of the waveforms (the P wave, the P-Q wave, the QRS wave, the S-T wave, the T wave, and the U wave) that can be identified through the electrocardiogram may change in various manners depending on the health condition. In other words, by analyzing the order of appearance, shapes, sizes, and intervals of the waveforms included in the electrocardiogram, a health condition can be inferred, and even the various causes that cause such a health condition can be inferred.

Meanwhile, an artificial neural network (ANN) is one of the detailed methodologies of machine learning, and refers to an algorithm that has a network form in which neurons, which are basic computational units corresponding to human nerve cells, are linked to one another. Representative artificial neural networks (ANNs) include a multilayer perceptron (MLP), a recurrent neural network (RNN), and a convolutional neural network (CNN).

More specifically, a multilayer perceptron (MLP) is an artificial neural network (ANN) that has a plurality of hidden layers between an input layer and an output layer. A recurrent neural network (RNN) is an artificial neural network (ANN) that has a recursive connection structure in which the output of neurons is input back to the input layer. Furthermore, a convolutional neural network (CNN) is an artificial neural network (ANN) that mimics the human optic nerve structure, and extracts a feature map from a plurality of convolutional layers, reduces the dimension of a matrix through subsampling, and extracts only important parts from the feature map.

On the other hand, a menu (or a diet) is a plan related to the types, amounts, and order of foods taken by a person during a meal. In the process of determining a menu, not only a person's preference but also the nutrients required according to the person's health condition should be taken into consideration. Accordingly, conventional services that recommend or suggest foods or a menu consider only the nutrients required according to the general health condition based on static factors such as a user's height, weight, and obesity level.

However, the nutrients required according to the health condition may change from moment to moment depending on the type and intensity of the activity being performed or performed immediately before, the types and amounts of foods taken immediately before, and/or the like. For example, a person who has just finished high-intensity exercise will require macronutrients such as protein and water, and a person who has experienced sudden diarrhea or vomiting will require micronutrients such as minerals. Furthermore, it is not practical for non-medical persons to periodically draw blood directly to determine their health conditions and required nutrients in their daily lives.

Therefore, there is a need for a means that can immediately recommend a menu suitable for a user's real-time health condition without requiring the user to take one or more burdensome actions.

## Disclosure

**Technical Problem**

One object of the present invention is to propose a method of recommending a menu suitable for a user's real-time health condition based on an electrocardiogram.

Another object of the present invention is to propose a computer program recorded on a storage medium in order to execute a method of recommending a menu suitable for a user's real-time health condition based on an electrocardiogram.

The objects of the present invention are not limited to the objects mentioned above, and other objects not mentioned will be clearly understood by those skilled in the art from the following description.

**Technical Solution**

In order to accomplish the above-described object, the present invention proposes a method of recommending a menu suitable for a user's real-time health condition based on an electrocardiogram. The menu recommendation method may include: obtaining an electrocardiogram signal measured for a user; identifying one or more lacked nutrients and one or more surplus nutrients corresponding to the user's health condition by analyzing the electrocardiogram signal using pre-trained artificial intelligence (AI); and setting a menu including at least one food that can supplement the identified lacked nutrients and avoid the identified surplus nutrients.

The menu recommendation method may further include, after setting the menu, transmitting information about the set menu to user equipment set in advance in accordance with the user.

More specifically, the identifying may include: segmenting the electrocardiogram signal into a plurality of segmentation signals according to a time-series order; inputting the plurality of segmentation signals to the artificial intelligence, and obtaining a plurality of probability values from the artificial intelligence; and determining whether nutrients are lacked, normal, or surplus based on the plurality of obtained probability values.

According to one embodiment, the segmenting into a plurality of segmentation signals may include segmenting the electrocardiogram signal into a plurality of segmentation signals each having a size corresponding to the size of a window while moving the window of a preset size along a time axis.

Meanwhile, the artificial intelligence may be configured to include a first artificial neural network (ANN) for electrolytes, a second artificial neural network (ANN) for proteins, and a third artificial neural network (ANN) for water. In this case, the determining may include determining whether electrolytes are lacked, normal, or surplus based on a plurality of probability values obtained from the first ANN, determining whether proteins are lacked, normal, or surplus based on a plurality of probability values obtained from the second ANN, and determining whether water is lacked, normal, or surplus based on a plurality of probability values obtained from the third ANN.

Furthermore, each of the hidden layers of the first to third artificial neural networks (ANNs) may include a generalized matrix factorization (GMF) layer for the learning of the linearity relation present between the features of the electrocardiogram signal and contents of nutrients and a multi-layer perceptron (MLP) for the learning of the non-linearity relation present between features of the electrocardiogram signal and contents of nutrients.

The determining may include determining whether the nutrients are lacked, normal, or surplus by using one of soft voting results based on the average value of the plurality of probability values from which outliers have been removed and hard voting results based on the majority of the plurality of probability values from which outliers have been removed.

The setting a menu may include: identifying two or more foods that do not contain the surplus nutrients or contain only threshold contents or less set in advance, and contain the identified lacked nutrients from a preset food dictionary; identifying a human weight set in advance in accordance with the user and a time weight corresponding to a time point at which the electrocardiogram signal was measured; and selecting one food from among the two or more identified foods by reflecting the human weight and the time weight therein.

Furthermore, the identifying may include determining that a warning is required for the user when a first time and a second time have a difference within a preset minimum biotransformation time and surplus nutrients identified based on an electrocardiogram signal measured for the user at the first time and lacked nutrients identified based on an electrocardiogram signal measured for the user at the second time are identical to each other.

In order to accomplish the above-described object, the present invention proposes a computer program recorded on a storage medium in order to execute a method of recommending a menu suitable for a user's real-time health condition based on an electrocardiogram. The computer program may be coupled to a computing device including: memory; a transceiver; an input/output device; and a processor for processing instructions loaded into the memory. Furthermore, the computer program may be a computer program recorded on a storage medium in order to execute obtaining, by the processor, an electrocardiogram signal measured for a user, identifying, by the processor, one or more lacked nutrients and one or more surplus nutrients corresponding to the user's health condition by analyzing the electrocardiogram signal using pre-trained artificial intelligence, and, setting, by the processor, a menu including at least one food that can supplement the identified lacked nutrients and avoid the identified surplus nutrients.

Specific details of other embodiments are included in the detailed description and the drawings.

**Advantageous Effects**

According to embodiments of the present invention, a menu optimized for a user's health condition at that time may be recommended simply by the user measuring an electrocardiogram.
The effects of the present invention are not limited to the effect mentioned above, and other effects not mentioned will be clearly understood by those skilled in the art, to which the present invention pertains, from the description of the claims.

**Description of Drawings**

FIG. 1 is an exemplary diagram illustrating a menu recommendation means according to one embodiment of the present invention;
FIG. 2 is an exemplary diagram illustrating a menu recommendation system according to one embodiment of the present invention;
FIG. 3 is a diagram of the logical configuration of a menu recommendation server according to one embodiment of the present invention;
FIG. 4 is an exemplary diagram illustrating an artificial neural network (ANN) according to one embodiment of the present invention;
FIGS. 5 and 6 are exemplary diagrams illustrating processes of segmenting an electrocardiogram signal according to some embodiments of the present invention;
FIGS. 7 and 8 are exemplary diagrams illustrating processes of determining whether a nutrient is lacked, normal, or surplus according to some embodiments of the present invention;
FIG. 9 is a diagram of the hardware configuration of a menu recommendation server according to one embodiment of the present invention; and
FIG. 10 is a flowchart illustrating a menu recommendation method according to one embodiment of the present invention.

**Mode for Invention**

It should be noted that the technical terms used herein are used only to describe specific embodiments and are not intended to limit the present invention. Furthermore, unless specifically defined otherwise herein, the technical terms used herein should be interpreted as having meanings generally understood by a person having ordinary skill in the art to which the present invention pertains, but should not be interpreted in an excessively comprehensive or excessively narrow sense. Furthermore, when the technical terms used herein are incorrect technical terms that do not accurately represent the spirit of the present invention, they should be replaced with and understood as technical terms that can be correctly understood by a person skilled in the art. Furthermore, the general terms used herein should be interpreted according to the definitions of dictionaries or according to the context, but should not be interpreted in an excessively narrow sense.
In addition, the singular expressions used herein include plural expressions unless the context clearly indicates otherwise. In the present application, the terms "include" or "have" should not be construed as necessarily including all of the various components or various steps described herein, and some of the components or steps may not be included or one or more additional components or steps may be included.
In addition, the terms including ordinal numbers, such as first, second, etc., used herein may be used to describe various components, but the components should not be limited by the terms. The terms are each used only for the purpose of distinguishing one component from another. For example, without departing from the scope of the present invention, the first component may be named the second component, and similarly, the second component may also be named the first component.
When a component is referred to as being "connected" or "coupled" to another component, it may be directly connected or coupled to the other component, but there may be another component therebetween. In contrast, when a component is referred to as being "directly connected" or "directly coupled" to another component, it should be understood that there are no other component therebetween.
Preferred embodiments according to the present invention will be described in detail with reference to the accompanying drawings below. Regardless of drawing numbers, identical or similar components will be given the same reference numerals and redundant descriptions thereof will be omitted. Furthermore, in the description of the present invention, when it is determined that a detailed description of a related known technology may obscure the gist of the present invention, the detailed description will be omitted. Furthermore, it should be noted that the accompanying drawings are only intended to facilitate the easy understanding of the spirit of the present invention and the spirit of the present invention should not be construed as being limited by the accompanying drawings. The spirit of the present invention should be construed as extending to all modifications, equivalents, and substitutes as well as the accompanying drawings.

As described above, conventional services that recommend or suggest foods or menus only take into consideration the nutrients required according to a general health condition based on static factors such as a user's height, weight, obesity level, and/or the like. However, the nutrients required according to the health condition may change from moment to moment depending on the type and intensity of the activity being performed or performed immediately before, the types and amounts of foods taken immediately before, and/or the like.

In order to overcome these limitations, the present invention proposes a means for recommending a menu suitable for a user's real-time health condition.

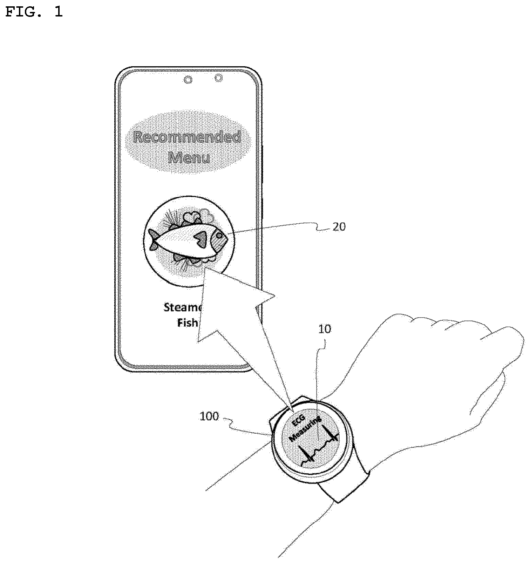

FIG. 1 is an exemplary diagram illustrating a menu recommendation means according to one embodiment of the present invention.

As shown in FIG. 1, the menu recommendation means according to one embodiment of the present invention may measure a user's electrocardiogram 10 using an electrocardiogram reader 100, may analyze the user's measured electrocardiogram 10 using artificial intelligence, may identify the nutrients required according to the user's real-time health condition, and may immediately recommend a menu 20 based on the identified nutrients to the user.

Accordingly, according to one embodiment of the present invention, the user may receive a recommendation for a menu optimized for the user's health condition at that time simply by measuring the electrocardiogram 10.

Apparatuses and methods for implementing the above-described features will be specifically described below.

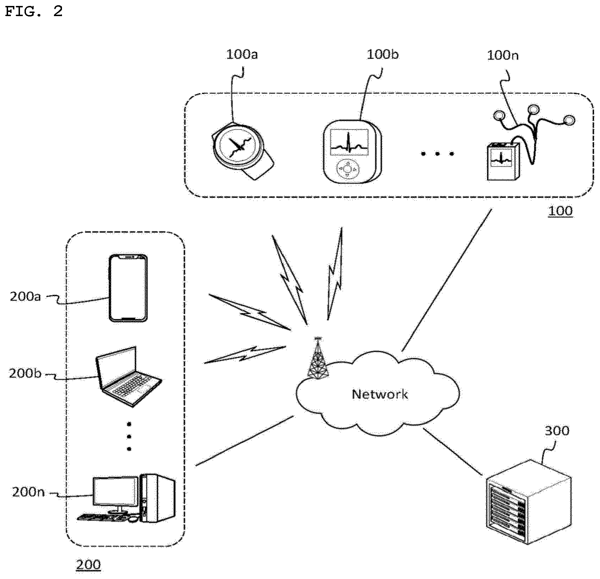

FIG. 2 is an exemplary diagram illustrating a menu recommendation system according to one embodiment of the present invention.

As shown in FIG. 2, the menu recommendation system according to the one embodiment of the present invention may be configured to include one or more electrocardiogram readers 100a, 100b, ⋯, and 100n (100), one or more pieces of user equipment 200a, 200b, ⋯, and 200n (200), and a menu recommendation server 300.

The components of the menu recommendation system according to the one embodiment of the present invention are merely functionally distinct components, so that two or more components may be implemented as an integrated component in an actual physical environment or one component may be implemented as separate components in an actual physical environment.

As for the individual components, each of the electrocardiogram readers 100 is a device capable of measuring and recording a user's electrocardiogram 10.

More specifically, each of the electrocardiogram readers 100 may induce changes in electric potential attributable to the electrical activity of the heart muscles via electrodes in contact with the user's body, may amplify the induced changes in electric potential, and may record them as waves.

The electrocardiogram reader 100 according to one embodiment of the present invention may induce an electrocardiogram according to any one of standard limb leads, unipolar limb leads, and precordial leads. The electrocardiogram (10) induction method of the electrocardiogram reader 100 is not limited thereto. The electrocardiogram reader 100 may be equipped with electrodes for a 1 lead, 6 leads, or 12 leads, and the number of leads of the electrocardiogram reader 100 is not limited thereto. Furthermore, the electrocardiogram reader 100 may have any one of the watch type 100a, the portable type 100b, and the holter type 100n, and the type of electrocardiogram reader 100 is not limited thereto.

An electrocardiogram signal (i.e., a recorded waveform signal) measured by the electrocardiogram reader 100 may be directly transmitted to the menu recommendation server 300 through the network function of the electrocardiogram reader 100 itself, or may be input to the menu recommendation server 300 via a third means. For example, the third means may be any one of a removable medium, the user equipment 200, and a data input action performed by a user, but is not limited thereto.

As the next component, each of the pieces of user equipment 200 may output information about a menu set by the menu recommendation server 300.

More specifically, the user equipment 200 may receive information about a menu from the menu recommendation server 300. Then, the user equipment 200 may output the received information about a menu.

The user equipment 200 according to one embodiment of the present invention is not limited to user equipment (UE) defined by the 3rd Generation Partnership Project (3GPP) or a mobile station (MS) defined by the Institute of Electrical and Electronics Engineers (IEEE). Any device may be accepted as the user equipment 200 as long as the device can transmit and receive data to and from the menu recommendation server 300 and perform operations based on the transmitted and received data.

For example, the user equipment 100 may be, but is not limited to, any one of fixed computing devices such as a desktop 200c, a workstation, and a server, and mobile computing devices such as a smartphone 200a, a laptop 200b, a tablet, a phablet, a portable multimedia player (PMP), a personal digital assistant PDA, and an e-book reader.

As the next component, the menu recommendation server 300 may identify the nutrients required according to a user's real-time health condition based on the electrocardiogram 10 measured by the electrocardiogram reader 100, and may transmit information about a menu 20 based on the identified nutrients to the user equipment 200.

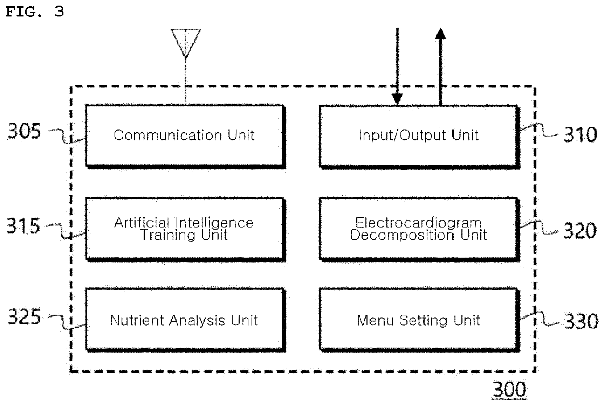

The specific components and operations of the menu recommendation server 300 will be described later with reference to FIGS. 3 to 10.

The one or more electrocardiogram readers 100, the one or more pieces of user equipment 200, and the menu recommendation server 300 that constitute the menu recommendation system described above may transmit and receive data using a network, formed by combining one or more of a secure line, a public wired communication network, and a mobile communication network, that directly connects the devices.

For example, the public wired communication network may include, but is not limited to, Ethernet, Digital Subscriber Line (xDSL), Hybrid Fiber Coax (HFC), and Fiber To The Home (FTTH) networks. Furthermore, the mobile communication network may include, but is not limited to, Code Division Multiple Access (CDMA), Wideband CDMA (WCDMA), High Speed Packet Access (HSPA), Long Term Evolution (LTE), and 5th generation mobile telecommunication networks.

The components of the menu recommendation server 300 having the features described above will be specifically described below.

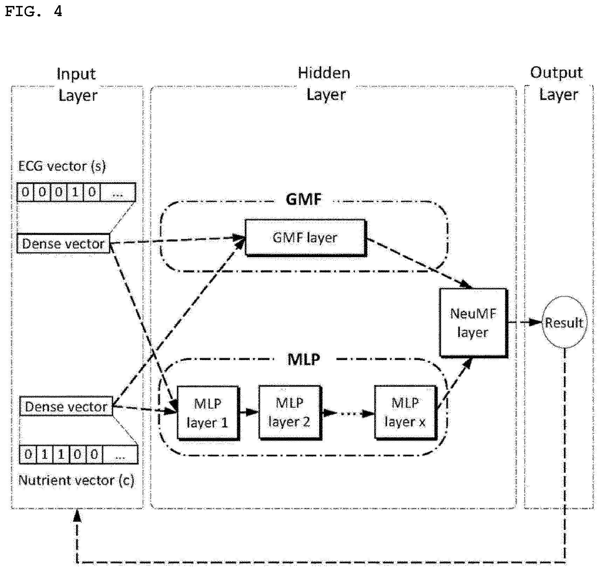

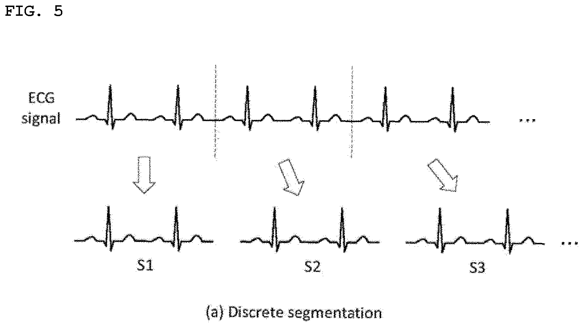

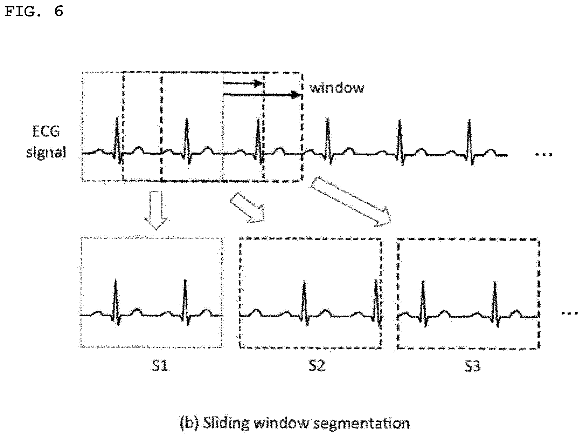

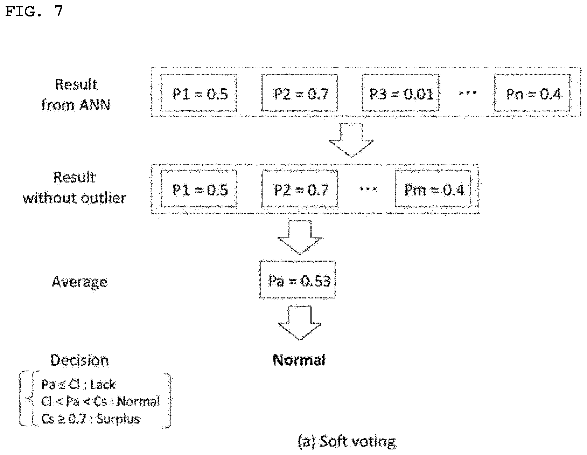

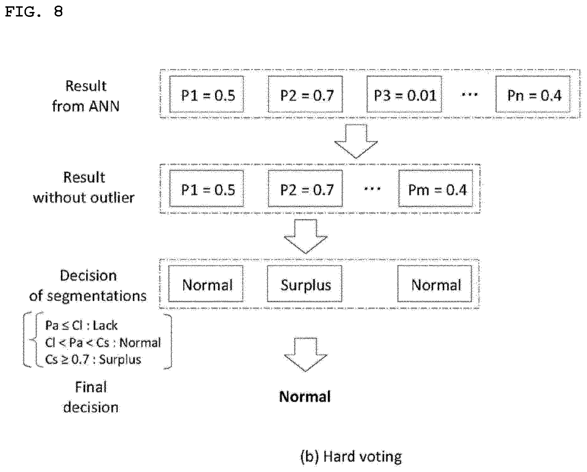

FIG. 3 is a diagram of the logical configuration of a menu recommendation server according to one embodiment of the present invention. FIG. 4 is an exemplary diagram illustrating an artificial neural network (ANN) according to one embodiment of the present invention. FIGS. 5 and 6 are exemplary diagrams illustrating processes of segmenting an electrocardiogram signal according to some embodiments of the present invention. Furthermore, FIGS. 7 and 8 are exemplary diagrams illustrating processes of determining whether a nutrient is lacked, normal, or surplus according to some embodiments of the present invention.

As shown in FIG. 3, the menu recommendation server 300 according to one embodiment of the present invention may be configured to include a communication unit 305, an input/output unit 310, an artificial intelligence training unit 315, an electrocardiogram decomposition unit 320, a nutrient analysis unit 325, and a menu setting unit 330.

The components of the menu recommendation server 300 according to the one embodiment of the present invention are merely functionally distinct components, so that two or more components may be implemented as an integrated component in an actual physical environment or one component may be implemented as separate components in an actual physical environment.

As for the individual components, the communication unit 305 may transmit and receive data to and from the electrocardiogram reader 100 and the user equipment 200.

More specifically, the communication unit 305 may receive an electrocardiogram (10) signal from the electrocardiogram reader 100. Furthermore, the communication unit 305 may transmit information about a menu 20 to the user equipment 200.

As the next component, the input/output unit 310 may receive commands from an administrator or output operation results via a user interface (UI).

More specifically, the input/output unit 310 may receive the size of a window, which is a criterion for the decomposition of an electrocardiogram (10) signal. The input/output unit 310 may receive a validated range, which is a criterion for the removal of outliers from probability values obtained from artificial intelligence. The input/output unit 310 may receive a food dictionary in which foods and nutrients are organized by matching each other. The input/output unit 310 may receive a threshold content, which is a boundary value at which additional intake is allowed for each surplus nutrient. The input/output unit 310 may receive a minimum biotransformation time, which is a criterion for determining that a special situation has occurred to a user. Furthermore, the input/output unit 310 may receive a threshold distance, which is a criterion range for the provision of information about restaurants.

Meanwhile, the input/output unit 310 may receive an activation function to be applied to the GMF layer of an artificial neural network (ANN), an activation function to be applied to the MLP layer thereof, a weight, and a bias.

Furthermore, the input/output unit 310 may output a user's electrocardiogram (10) signal. The input/output unit 310 may also output information about a menu 20 to be recommended to the user.

As the next component, the artificial intelligence training unit 315 may train an artificial intelligence for identifying one or more lacked and/or surplus nutrients corresponding to a user's health condition based on an electrocardiogram (10) signal.

More specifically, the artificial intelligence, which is the training target of the artificial intelligence training unit 315, may be configured to include a first ANN for electrolytes, a second ANN for proteins, and a third ANN for water.

The artificial intelligence training unit 315 may train the first ANN based on data on the features of the electrocardiogram signal and contents of electrolytes input by an administrator. The artificial intelligence training unit 315 may train the second ANN based on data on the features of the electrocardiogram signal and the contents of the proteins input by the administrator. Furthermore, the artificial intelligence training unit 315 may train the third ANN based on data on the features of the electrocardiogram signal and content of water input by the administrator.

In this case, the electrolytes are substances that dissolve in a solvent and ionize to generate positive and negative ions. The electrolytes according to one embodiment of the present invention may include sodium (Na), potassium (Ka), and calcium (Ca), but are not limited thereto. The proteins are organic substances formed by combining amino acids. The proteins according to one embodiment of the present invention may include albumin, but are not limited thereto.

Each of the first ANN, the second ANN, and the third ANN, which are the training targets of the artificial intelligence training unit 315, may be configured to include an input layer, a hidden layer, and an output layer.

In particular, as shown in FIG. 4, the hidden layers of the first ANN to the third ANN according to one embodiment of the present invention may each be configured in a form in which a generalized matrix factorization (GMF) and a multi-layer perceptron (MLP) are mixed.

For the convenience of description, all or part of the first ANN to the third ANN may be simply described as the ANN.

In each of the ANNs according to one embodiment of the present invention, the GMF layer is a layer for the learning of the linearity relation present between the features of the electrocardiogram signal and the contents of nutrients, and may be implemented using Equation 1 below:

$$a\left(h^T(p_u \odot q_i)\right) \tag{1}$$

where $p_u$ is the dense vector of the features of the electrocardiogram signal, $q_i$ is the dense vector of the content of the nutrients, $p_u \odot q_i$ is the Hadamard product between the dense vector of the features of the electrocardiogram signal and the dense vector of the contents of the nutrients, $a$ is the activation function of the ANN, $h$ is the edge weights of the hidden layers, and $h^T$ is the vector product of the edge weights.

A dense vector is a vector whose dimension is reduced by embedding a vector represented by one-hot encoding. In other words, a dense vector is a vector whose dimension is reduced by removing values of 0 from a sparse vector represented by one-hot encoding.

Furthermore, in each of the ANNs, the MLP layer is a layer for the learning of the non-linearity relation present between the features of the electrocardiogram signal and the contents of nutrients, and may be implemented using Equations 2 and 3 below:

$$\varphi_2(z_1) = a_2(W_2^T z_1 + b_2) \tag{2}$$

$$\sigma\left(h^T \varphi_L(z_{L-1})\right) \tag{3}$$

where $p_u$ is the dense vector of the features of the electrocardiogram signal, $q_i$ is the dense vector of the content of the nutrients, z is a concatenation of the dense vector of the features of the electrocardiogram signal and the dense vector of the content of the nutrients, $a$ is the activation function of the ANN, $W$ is the weight of each layer constituting the MLP, $W^T$ is the vector product of the weights, $b$ is the bias of each layer constituting the MLP, L is the number of layers constituting the hidden layer, and $\sigma$ is the sum of the progression.

Furthermore, each of the ANNs may be configured in the form of a recurrent neural network having a recursive connection structure in which the probability value output to the output layer is added to the vector for the contents of nutrients and then re-input to the input layer.

In this manner, each of the ANNs of the artificial intelligence training unit 315 has a form in which a GMF and an MLP are mixed, so that not only the linearity relation between the features of the electrocardiogram signal and the contents of nutrients but also the nonlinearity relation can be machine-learned at the same time.

Referring back to FIG. 3 to describe the next component, the electrocardiogram decomposition unit 320 may decompose the electrocardiogram (10) signal measured by the electrocardiogram reader 100.

More specifically, the electrocardiogram decomposition unit 320 may obtain the electrocardiogram (10) signal measured for the user. That is, the electrocardiogram decomposition unit 320 may directly receive the electrocardiogram (10) signal from the electrocardiogram reader 100 via the communication unit 305, or may directly receive the electrocardiogram (10) signal via the input/output unit 310.

The electrocardiogram decomposition unit 320 may segment the electrocardiogram (10) signal into a plurality of segmentation signals according to the time-series order measured by the user.

Basically, as shown in FIG. 5, the electrocardiogram decomposition unit 320 may segment the electrocardiogram (10) signal into a plurality of segmentation signals S1, S2, S3, ... by discretely cutting the electrocardiogram (10) signal at a predetermined size without overlap areas. In this case, the size for the discrete cutting of the electrocardiogram (10) signal may be a specific value set in advance by the input/output unit 310, but may not be limited thereto and may be a randomized value.

In contrast, as shown in FIG. 6, the electrocardiogram decomposition unit 320 may segment the electrocardiogram (10) signal into a plurality of segmentation signals S1, S2, S3, ... having a size corresponding to that of the window by cutting the electrocardiogram (10) signal while sliding the window through the electrocardiogram (10) signal along the time axis. In this case, the size of the window for the cutting of the electrocardiogram (10) signal may be a specific value set in advance by the input/output unit 310.

Meanwhile, the electrocardiogram (10) signal may include the noise generated by the user's behavior during a measurement process. Accordingly, the electrocardiogram decomposition unit 320 may remove the noise included in the plurality of segmentation signals S1, S2, S3, ....

More specifically, the electrocardiogram decomposition unit 320 may identify the displacements of the P wave, P-Q wave, QRS wave, S-T wave, and T wave included in each of the plurality of segmentation signals S1, S2, S3, .... The electrocardiogram decomposition unit 320 may generate a normal distribution of the displacements of the identified P wave, P-Q wave, QRS wave, S-T wave, and T wave. Furthermore, the electrocardiogram decomposition unit 320 may selectively remove only segmentation signals, having the displacements of the P wave, P-Q wave, QRS wave, S-T wave, or T wave included in a preset noise range, from the generated normal distribution.

Referring back to FIG. 3 to describe the next component, the nutrient analysis unit 325 may identify one or more lacked and surplus nutrients corresponding to the user's health condition by analyzing the electrocardiogram (10) signal by using artificial intelligence.

In this case, a lacked nutrient is a nutrient that requires immediate intake in a large quantity in light of a user's health condition. A surplus nutrient is a nutrient that does not cause a health problem even when it is not taken immediately, in light of the user's health condition. Furthermore, a normal nutrient is a nutrient that requires intake in the amount required to maintain daily life in light of the user's health condition.

First, the nutrient analysis unit 325 may obtain a plurality of probability values P1, P2, P3, ..., Pn from the artificial intelligence by inputting the plurality of segmentation signals S1, S2, S3, ..., obtained through the segmentation of the electrocardiogram decomposition unit 320, to the artificial intelligence.

The artificial intelligence according to one embodiment of the present invention is configured to include the first ANN to the third ANN, so that the nutrient analysis unit 325 may input the plurality of segmentation signals S1, S2, S3, ... to each of the first ANN to the third ANN.

More specifically, the electrocardiogram decomposition unit 320 may extract one or more features based on the displacements of the P wave, the P-Q wave, the QRS wave, the S-T wave, and the T wave included in each of the plurality of segmentation signals S1, S2, S3, ... according to the feature extraction criteria set in advance. The electrocardiogram decomposition unit 320 may generate a vector of the features of the electrocardiogram signal by one-hot encoding the extracted one or more features. The electrocardiogram decomposition unit 320 may generate a dense vector having a reduced dimension by embedding the generated vector of the features of the electrocardiogram signal. The electrocardiogram decomposition unit 320 may input the generated dense vector to the GMF and MLP layers of each of the first ANN to the third ANN. Furthermore, the electrocardiogram decomposition unit 320 may obtain a plurality of probability values P1, P2, P3, ..., Pn corresponding to the plurality of segmentation signals S1, S2, S3, ... from each of the first ANN to the third ANN.

Thereafter, the nutrient analysis unit 325 may determine whether the individual nutrients are lacked, normal, or surplus based on the plurality of obtained probability values P1, P2, P3, ..., Pn.

More specifically, the nutrient analysis unit 325 may determine whether electrolytes are lacked, normal, or surplus based on the plurality of probability values obtained from the first ANN. The nutrient analysis unit 325 may determine whether proteins are lacked, normal, or surplus based on the plurality of probability values obtained from the second ANN. Furthermore, the nutrient analysis unit 325 may determine whether water is lacked, normal, or surplus based on the plurality of probability values obtained from the third ANN.

As shown in FIGS. 7 and 8, the nutrient analysis unit 325 may remove outliers, included in the plurality of probability values P1, P2, P3, ..., Pn obtained from each of the ANNs, based on a preset validated range. The nutrient analysis unit 325 may determine whether nutrients are lacked, normal, or surplus by using soft voting results based on the average value Pa of the plurality of probability values P1, P2, ..., Pm from which the outliers have been removed or by using hard voting results based on the majority of the plurality of probability values P1, P2, ..., Pm from which the outliers have been removed.

In this case, for the soft voting, the nutrient analysis unit 325 may calculate the average value Pa of the plurality of probability values P1, P2, ..., Pm from which outliers have been removed. Then, the nutrient analysis unit 325 may determine the nutrients to be lacked when the calculated average value Pa is smaller than or equal to a preset lack criterion Cl, may determine the nutrients to be surplus when the calculated average value Pa is equal to or larger than a surplus criterion Cs, and may determine the nutrients to be normal when the calculated average value Pa is larger than the lack criterion Cl and smaller than the surplus criterion Cs.

In contrast, for the hard voting, the nutrient analysis unit 325 may determine whether each of the plurality of probability values P1, P2, ..., Pm from which outliers have been removed is lacked, normal, or surplus. That is, the nutrient analysis unit 325 may determine the nutrients to be lacked when each probability value Px is smaller than or equal to the lack criterion Cl, may determine the nutrients to be surplus when each probability value Px is larger than or equal to the surplus criterion Cs, and may determine the nutrient to be normal when each probability value Px is larger than the lack criterion Cl and smaller than the surplus criterion Cs. Furthermore, the nutrient analysis unit 325 may determine the majority of the plurality of determination results to be a final result.

Meanwhile, the nutrient analysis unit 325 may receive electrocardiogram (10) signals measured at a first time and a second time, respectively, that have a difference in time within the minimum biotransformation time set in advance for the user. When the surplus nutrients identified based on the electrocardiogram (10) signal measured at the first time for the user are the same as the lacked nutrients identified based on the electrocardiogram (10) signal measured at the second time for the user, the nutrient analysis unit 325 may determine that a warning is required for the user. When it is determined that a warning is required for the user, the nutrient analysis unit 325 may transmit a message providing notification that an abnormality has occurred in the body to the user equipment 200 set in accordance with the user.

Referring back to FIG. 3 to describe the next component, the menu setting unit 330 may set a menu including one or more foods that can supplement the lacked nutrients and avoid the surplus nutrients identified via the nutrient analysis unit 325.

More specifically, the menu setting unit 330 may identify two or more foods that do not contain surplus nutrients or contain only surplus nutrients of threshold contents or less set in advance and contain lacked nutrients from a preset food dictionary based on information about one or more lacked nutrients, surplus nutrients, and normal nutrients identified via the nutrient analysis unit 325.

The menu setting unit 330 may identify a human weight set in advance in accordance with the user and a time weight corresponding to the time at which the electrocardiogram (10) signal was measured.

In this case, the human weight may be a value set in accordance with the types of lacked and surplus nutrients identified based on the electrocardiogram (10) signals measured for the user and a person set as a cohabitant. Furthermore, the time weight may be a value set in accordance with the type of season or meal time corresponding to the time at which the electrocardiogram (10) signal was measured for the user.

The menu setting unit 330 may select one food from among two or more identified foods by reflecting the human weight and the time weight therein. Furthermore, the menu setting unit 330 may identify at least one menu template including the selected one food, and may set a menu using the identified at least one menu template. In this case, the menu templates may be a list configured to include two or more foods that are set in advance based on the food compatibility between the foods.

When setting a menu using at least one food template in this manner, the menu setting unit 330 may set a menu by further including therein information about the intake order and recommended intake amounts of ingredients included in one or more foods included in the food template. Furthermore, the menu setting unit 330 may also set a menu by further including therein information about restaurants, which are present within a threshold distance set in advance from the user equipment 200 and can provide one or more foods included in the menu, based on the location information of the user equipment 200.

In addition, the menu setting unit 330 may transmit information about the set menu to the user equipment 200 set in advance in accordance with the user.

Hardware for implementing the logical components of the menu recommendation server 300 having the features described above will be described in more detail below.

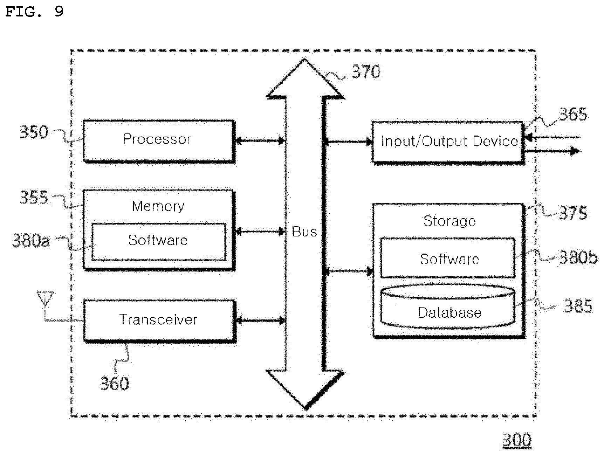

FIG. 9 is a diagram of the hardware configuration of a menu recommendation server according to one embodiment of the present invention.

As shown in FIG. 9, the menu recommendation server 300 according to the one embodiment of the present invention may be configured to include a processor 350, memory 355, a transceiver 360, an input/output device 365, a data bus 370, and storage 375.

More specifically, the processor 350 may implement the operations and functions of the menu recommendation server 300 based on instructions according to software 380a implementing a menu recommendation method that has been loaded into the memory 355.

The memory 355 may be loaded with software 380b implementing the menu recommendation method that is stored in the storage 375.

The transceiver 360 may transmit and receive data to and from one or more of the electrocardiogram reader 100 and the user equipment 200.

The input/output device 365 may receive the signals required for the operation of the menu recommendation server 300 or output operation results to the outside according to the commands of the processor 350.

The data bus 370 may be connected to the processor 350, the memory 355, the transceiver 360, the input/output device 365, and the storage 375, and may serve as a passage for the transmission of signals between the individual components.

The storage 375 may store application programming interfaces (APIs), library files, and resource files required for executing the software 380a implementing menu recommendation methods according to various embodiments of the present invention. The storage 375 may store the software 380b implementing the menu recommendation methods according to the various embodiments of the present invention. Furthermore, the storage 375 may include a database 385 for storing artificial intelligence training data, a food dictionary, menu templates, and various setting values.

According to one embodiment of the present invention, the software 380a and 380b for implementing the menu recommendation method loaded into the memory 355 or stored in the storage 375 may be a computer program stored on a storage medium in order to execute the step of obtaining, by the processor 350, an electrocardiogram (10) signal measured for a user via the transceiver 360 or input/output device 365, the step of identifying, by the processor 350, one or more lacked nutrients and one or more surplus nutrients corresponding to the user's health condition by analyzing the electrocardiogram (10) signal using pre-trained artificial intelligence, and the step of setting, by the processor 350, a menu including at least one food that can supplement the identified lacked nutrients and avoid the identified surplus nutrients.

More specifically, the processor 350 may be configured to include one or more of a Central Processing Unit (CPU), an Application-Specific Integrated Circuit (ASIC), a chipset, and a logic circuit, but is not limited thereto.

The memory 355 may be configured to include one or more of Read-Only Memory (ROM), Random Access Memory (RAM), flash memory, and a memory card, but is not limited thereto.

The input/output device 365 may be configured to include one or more of input devices such as buttons, switches, a keyboard, a mouse, a joystick, and a touch screen, and one or more of output devices such as a liquid crystal display (LCD), a light emitting diode (LED) display, an organic light emitting diode (OLED) display, an active matrix OLED (AMOLED) display, a printer, and a plotter, but is not limited thereto.

When the embodiments included herein are implemented as software, the above-described method may be implemented as modules (processes, functions, and/or the like) that perform the above-described functions. The individual modules may be loaded into the memory 355 and executed by the processor 350. The memory 355 may be present inside or outside the processor 350, and may be connected to the processor 350 via various means known to the public.

The individual components shown in FIG. 9 may be implemented by various means (e.g., hardware, firmware, software, or a combination thereof). When implemented by hardware, an embodiment of the present invention may be implemented by one or more Application Specific Integrated Circuits (ASICs), Digital Signal Processors (DSPs), Digital Signal Processing Devices (DSPDs), Programmable Logic Devices (PLDs), Field Programmable Gate Arrays (FPGAs), processors, controllers, microcontrollers, microprocessors, and/or the like.

Furthermore, when implemented by firmware or software, one embodiment of the present invention may be implemented in the form of modules, procedures, functions, and/or the like that perform the functions or operations described above, and may be recorded on a storage medium that can be read via various computer means. In this case, the storage medium may include program instructions, data files, data structures, and the like alone or in combination.

The program instructions recorded on the storage medium may be those specifically designed and configured for the present invention, or may be those known and available to those having ordinary knowledge in the computer software industry. For example, the storage medium includes magnetic media such as a hard disk, a floppy disk, and magnetic tape, optical media such as compact disk read-only memory (CD-ROM) and a digital video disk (DVD), magneto-optical media such as a floptical disk, and hardware devices specifically configured to store and execute program instructions such as ROM, RAM, and flash memory.

Examples of the program instructions may include not only machine language codes such as those that are generated by a compiler, but also high-level language codes that can be executed by a computer using an interpreter or the like. Such hardware devices may be configured to operate as one or more pieces of software to perform the operations of the present invention, and vice versa.

The operation of the menu recommendation server 300 described above will be specifically described below.

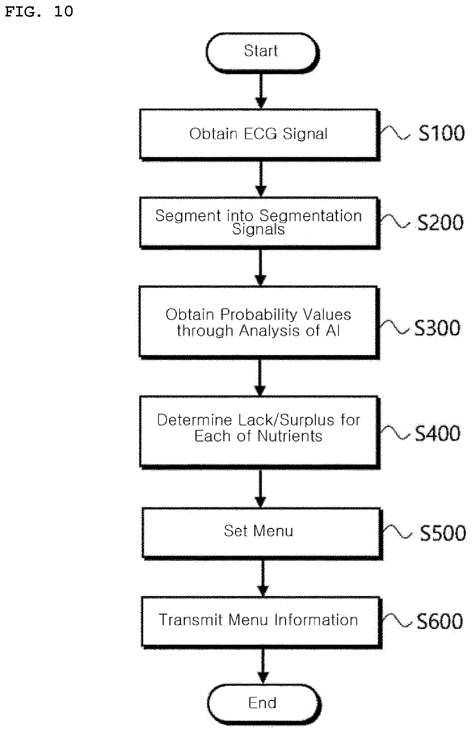

FIG. 10 is a flowchart illustrating a menu recommendation method according to one embodiment of the present invention.

As shown in FIG. 10, the menu recommendation server 300 according to one embodiment of the present invention may obtain an electrocardiogram (10) signal measured for a user in step S100.

More specifically, the menu recommendation server 300 may directly receive the electrocardiogram (10) signal from the electrocardiogram reader 100, or may directly receive a signal from a user or another person.

Thereafter, the menu recommendation server 300 may segment the electrocardiogram (10) signal into a plurality of segmentation signals S1, S2, S3, ... according to the time-series order measured by the user in step S200.

According to one embodiment, the menu recommendation server 300 may segment the electrocardiogram (10) signal into a plurality of segmentation signals S1, S2, S3, ... by discretely cutting the electrocardiogram (10) signal at a predetermined size without overlap areas. According to another embodiment, the menu recommendation server 300 may segment the electrocardiogram (10) signal into a plurality of segmentation signals S1, S2, S3, ... having a size corresponding to that of the window by cutting the electrocardiogram (10) signal while sliding the window through the electrocardiogram (10) signal along the time axis.

Meanwhile, the menu recommendation server 300 may remove the noise included in the plurality of segmentation signals S1, S2, S3, ....

Thereafter, the menu recommendation server 300 may obtain a plurality of probability values P1, P2, P3, ..., Pn by analyzing the plurality of segmentation signals S1, S2, S3, ... using the artificial intelligence in step S300.

More specifically, the menu recommendation server 300 may extract one or more features based on the displacements of the P wave, the P-Q wave, the QRS wave, the S-T wave, and the T wave included in each of the plurality of segmentation signals S1, S2, S3, ... according to the feature extraction criteria set in advance. The menu recommendation server 300 may generate a vector of the features of the electrocardiogram signal by one-hot encoding the extracted one or more features. The menu recommendation server 300 may generate a dense vector having a reduced dimension by embedding the generated vector of the features of the electrocardiogram signal. The menu recommendation server 300 may input the generated dense vector to the GMF and MLP layers of each of the first ANN to the third ANN. Furthermore, the menu recommendation server 300 may obtain a plurality of probability values P1, P2, P3, ..., Pn corresponding to the plurality of segmentation signals S1, S2, S3, ... from each of the first ANN to the third ANN.

Thereafter, the menu recommendation server 300 may determine whether each nutrient is lacked, normal, or surplus based on the plurality of obtained probability values P1, P2, P3, ..., Pn in step S400.

More specifically, the menu recommendation server 300 may determine whether electrolytes are lacked, normal, or surplus based on the plurality of probability values obtained from the first ANN. The menu recommendation server 300 may determine whether proteins are lacked, normal, or surplus based on the plurality of probability values obtained from the second ANN. Furthermore, the menu recommendation server 300 may determine whether water is lacked, normal, or surplus based on the plurality of probability values obtained from the third ANN.

To this end, the menu recommendation server 300 may remove outliers, included in the plurality of probability values P1, P2, P3, ..., Pn obtained from each of the ANNs, based on the preset validated range. The menu recommendation server 300 may determine whether nutrients are lacked, normal, or surplus by using soft voting results based on the average value $Pa$ of the plurality of probability values P1, P2, ..., Pm from which the outliers have been removed, or by using hard voting results based on the majority of the plurality of probability values P1, P2, ..., Pm from which the outliers have been removed.

Thereafter, the menu recommendation server 300 may set a menu including one or more foods that can supplement lacked nutrients and avoid surplus nutrients in step S500.

More specifically, the menu recommendation server 300 may identify two or more foods that do not contain surplus nutrients or contain only surplus nutrients of threshold contents or less set in advance and contain lacked nutrients from a preset food dictionary based on information about the identified lacked nutrients, surplus nutrients, and normal nutrients. The menu recommendation server 300 may identify a human weight set in advance in accordance with the user and a time weight corresponding to the time at which the electrocardiogram (10) signal was measured. The menu recommendation server 300 may select one food from among two or more identified foods by reflecting the human weight and the time weight therein. The menu recommendation server 300 may identify at least one menu template including the selected one food, and may set a menu using the identified at least one menu template.

Finally, the menu recommendation server 300 may transmit information about the set menu to the user equipment 200 set in advance in accordance with the user in step s600.

As described above, although the preferred embodiments of the present invention have been disclosed in the present specification and the accompanying drawings, it is obvious to those skilled in the art that other modified examples based on the technical spirit of the present invention can be implemented in addition to the embodiments disclosed herein. Furthermore, although specific terms have been used in the present specification and the accompanying drawings, they have been used only in general senses to easily describe the technical content of the present invention and to help the understanding of the present invention, and are not intended to limit the scope of the present invention. Therefore, the detailed description above should not be construed as restrictive and should be considered as exemplary in all aspects. The scope of the present invention should be determined by a reasonable interpretation of the appended claims, and all changes within the equivalent scope of the present invention are included in the scope of the present invention.

**Claims**

1. A menu recommendation method, comprising:

   obtaining an electrocardiogram signal measured for a user;
   identifying one or more lacked nutrients and one or more surplus nutrients corresponding to the user's health condition by analyzing the electrocardiogram signal using pre-trained artificial intelligence; and
   setting a menu including at least one food that can supplement the identified lacked nutrients and avoid the identified surplus nutrients.

2. The menu recommendation method of claim 1, wherein the identifying comprises:

   segmenting the electrocardiogram signal into a plurality of segmentation signals according to a time-series order;
   inputting the plurality of segmentation signals to the artificial intelligence, and obtaining a plurality of probability values from the artificial intelligence; and
   determining whether nutrients are lacked, normal, or surplus based on the plurality of obtained probability values.

3. The menu recommendation method of claim 2, wherein the segmenting into a plurality of segmentation signals comprises segmenting the electrocardiogram signal into a plurality of segmentation signals each having a size corresponding to a size of a window while moving the window of a preset size along a time axis.

4. The menu recommendation method of claim 2, wherein:

   the artificial intelligence is configured to include a first artificial neural network (ANN) for electrolytes, a second artificial neural network (ANN) for proteins, and a third artificial neural network (ANN) for water; and
   the determining comprises determining whether electrolytes are lacked, normal, or surplus based on a plurality of probability values obtained from the first ANN, determining whether proteins are lacked, normal, or surplus based on a plurality of probability values obtained from the second ANN, and determining whether water is lacked, normal, or surplus based on a plurality of probability values obtained from the third ANN.

5. The menu recommendation method of claim 4, wherein each of hidden layers of the first to third artificial neural networks (ANNs) comprises a generalized matrix factorization (GMF) layer for learning of a linearity relation present between the features of the electrocardiogram signal and contents of nutrients and a multi-layer perceptron (MLP) for learning of a non-linearity relation present between features of the electrocardiogram signal and contents of nutrients.

6. The menu recommendation method of claim 2, wherein the determining comprises determining whether the nutrients are lacked, normal, or surplus by using one of soft voting results based on an average value of the plurality of probability values from which outliers have been removed and hard voting results based on a majority of the plurality of probability values from which outliers have been removed.

7. The menu recommendation method of claim 1, further comprising, after the setting a menu, transmitting information about the set menu to user equipment set in advance in accordance with the user.

8. The menu recommendation method of claim 7, wherein the setting a menu comprises:

   identifying two or more foods that do not contain the surplus nutrients or contain only threshold contents or less set in advance, and contain the identified lacked nutrients from a preset food dictionary;
   identifying a human weight set in advance in accordance with the user and a time weight corresponding to a time point at which the electrocardiogram signal was measured; and
   selecting one food from among the two or more identified foods by reflecting the human weight and the time weight therein.

9. The menu recommendation method of claim 1, wherein the identifying comprises determining that a warning is required for the user when a first time and a second time have a difference within a preset minimum biotransformation time and surplus nutrients identified based on an electrocardiogram signal measured for the user at the first time and lacked nutrients identified based on an electrocardiogram signal measured for the user at the second time are identical to each other.

10. A computer program recorded on a storage medium, which is coupled to a computing device, including:

    memory;
    a transceiver;
    an input/output device; and
    a processor for processing instructions loaded into the memory, in order to execute:

       obtaining, by the processor, an electrocardiogram signal measured for a user;

identifying, by the processor, one or more lacked nutrients and one or more surplus nutrients corresponding to the user's health condition by analyzing the electrocardiogram signal using pre-trained artificial intelligence; and

setting, by the processor, a menu including at least one food that can supplement the identified lacked nutrients and avoid the identified surplus nutrients.

FIG. 1

EP 4 546 363 A1

FIG. 2

FIG. 3

| | |
|---|---|
| 305 — Communication Unit | Input/Output Unit — 310 |
| 315 — Artificial Intelligence Training Unit | Electrocardiogram Decomposition Unit — 320 |
| 325 — Nutrient Analysis Unit | Menu Setting Unit — 330 |

300

FIG. 4

FIG. 5

(a) Discrete segmentation

FIG. 6

(b) Sliding window segmentation

FIG. 7

Result from ANN | P1 = 0.5 | P2 = 0.7 | P3 = 0.01 | ··· | Pn = 0.4

Result without outlier | P1 = 0.5 | P2 = 0.7 | ··· | Pm = 0.4

Average | Pa = 0.53

Decision
$$Pa \leq Cl : Lack$$
$$Cl < Pa < Cs : Normal$$
$$Cs \geq 0.7 : Surplus$$

Normal

(a) Soft voting

FIG. 8

(b) Hard voting

FIG. 9

FIG. 10

```
                    ┌─────────────┐
                    │    Start    │
                    └──────┬──────┘
                           │
                           ▼
          ┌────────────────────────────────┐
          │       Obtain ECG Signal        │ ～ S100
          └────────────────┬───────────────┘
                           │
                           ▼
          ┌────────────────────────────────┐
          │   Segment into Segmentation    │ ～ S200
          │            Signals             │
          └────────────────┬───────────────┘
                           │
                           ▼
          ┌────────────────────────────────┐
          │     Obtain Probability Values  │ ～ S300
          │      through Analysis of AI    │
          └────────────────┬───────────────┘
                           │
                           ▼
          ┌────────────────────────────────┐
          │    Determine Lack/Surplus for  │ ～ S400
          │       Each of Nutrients        │
          └────────────────┬───────────────┘
                           │
                           ▼
          ┌────────────────────────────────┐
          │           Set Menu             │ ～ S500
          └────────────────┬───────────────┘
                           │
                           ▼
          ┌────────────────────────────────┐
          │    Transmit Menu Information    │ ～ S600
          └────────────────┬───────────────┘
                           │
                           ▼
                    ┌─────────────┐
                    │     End     │
                    └─────────────┘
```

## INTERNATIONAL SEARCH REPORT

| International application No. |
|---|
| **PCT/KR2023/010703** |

### A.   CLASSIFICATION OF SUBJECT MATTER

**G16H 20/60**(2018.01)i; **G16H 50/50**(2018.01)i; **G16H 50/20**(2018.01)i; **G16H 50/70**(2018.01)i; **A61B 5/346**(2021.01)i; **G06N 3/045**(2023.01)i; **G06N 3/042**(2023.01)i; **G06N 3/08**(2006.01)i

According to International Patent Classification (IPC) or to both national classification and IPC

### B.   FIELDS SEARCHED

Minimum documentation searched (classification system followed by classification symbols)

G16H 20/60(2018.01); A61B 5/00(2006.01); G06F 3/0481(2013.01); G06N 3/02(2006.01); G06Q 10/00(2006.01); G06Q 30/02(2012.01); G06Q 30/06(2012.01); G06Q 50/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 심전도(ElectroCardioGram, ECG), 인공지능(artificial intelligence, AI), 영양분 (nourishment), 식단(menu), 추천(recommendation), 인공신경망(artificial neural network, ANN)

### C.   DOCUMENTS CONSIDERED TO BE RELEVANT

| Category* | Citation of document, with indication, where appropriate, of the relevant passages | Relevant to claim No. |
|---|---|---|
| Y | KR 10-2022-0032678 A (SWALLABY) 15 March 2022 (2022-03-15)<br>See paragraphs [0046] and [0056] and claims 1-2 and 5-6. | 1-3,7,9,10 |
| A | | 4-6,8 |
| Y | KR 10-2019-0019397 A (CHUNGBUK PROVINCIAL COLLEGE INDUSTRY ACADEMIC COOPERATION FOUNDATION) 27 February 2019 (2019-02-27)<br>See paragraphs [0063]-[0068], [0104]-[0109] and [0111]-[0112], claims 1 and 3-4 and figures 3-11. | 1-3,7,9,10 |
| Y | KR 10-2021-0052122 A (SAMYANG CORPORATION) 10 May 2021 (2021-05-10)<br>See paragraphs [0057]-[0067] and [0084], claim 1 and figures 1-4. | 2,3,9 |
| Y | KR 10-2022-0091779 A (LG ELECTRONICS INC.) 01 July 2022 (2022-07-01)<br>See paragraphs [0019]-[0020], [0051] and [0121]-[0123], claims 1 and 7 and figures 1-4b. | 1,7,10 |

☑ Further documents are listed in the continuation of Box C.     ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **13 November 2023** | **13 November 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| | International application No. |
| --- | --- |
| | **PCT/KR2023/010703** |

**C.      DOCUMENTS CONSIDERED TO BE RELEVANT**

| Category* | Citation of document, with indication, where appropriate, of the relevant passages | Relevant to claim No. |
| --- | --- | --- |
| Y | JP 2009-009350 A (NEC CORP.) 15 January 2009 (2009-01-15)<br>See paragraphs [0030]-[0033] and claims 1 and 8. | 1,7,10 |

Form PCT/ISA/210 (second sheet) (July 2022)

INTERNATIONAL SEARCH REPORT
Information on patent family members

International application No.

**PCT/KR2023/010703**

| Patent document cited in search report | | | Publication date (day/month/year) | Patent family member(s) | | | Publication date (day/month/year) |
|---|---|---|---|---|---|---|---|
| KR | 10-2022-0032678 | A | 15 March 2022 | KR | 10-2548357 | B1 | 28 June 2023 |
| KR | 10-2019-0019397 | A | 27 February 2019 | KR | 10-2033065 | B1 | 16 October 2019 |
| KR | 10-2021-0052122 | A | 10 May 2021 | None | | | |
| KR | 10-2022-0091779 | A | 01 July 2022 | None | | | |
| JP | 2009-009350 | A | 15 January 2009 | None | | | |

Form PCT/ISA/210 (patent family annex) (July 2022)